# EUROPEAN PATENT APPLICATION

(11) **EP 2 106 789 A1**
(43) Date of publication of application: **07.10.2009**
(21) Application number: 08103277.3
(22) Date of filing: 31.03.2008
(51) Int. Cl.: A61K 9/20, A61K 31/4184

(54) **Pharmaceutical composition comprising candesartan**

(71) Applicant: KRKA, tovarna zdravil, d.d., Novo mesto, 8501 Novo mesto (SI)
(72) Inventor: Pilgaonkar, Pratibha Sudhir 221, Annexe Building, Goregaon-Mulund Link Road, Bhandup West, Mumbai 400 078 (IN); Rustomjee, Maharukh Tehmasp 221, Annexe Building, Goregaon-Mulund Link Road, Bhandup West, Mumbai 400 078 (IN); Gandh, Anilkumar Surendrakumar 221, Annexe Building, Goregaon-Mulund Link Road, Bhandup West, Mumbai 400 078 (IN); Bagde, Pradnya Mangesh 221, Annexe Building, Goregaon-Mulund Link Road, Bhandup West, Mumbai 400 078 (IN); Vrecer, Franc, 8351 Straza pri Novem Mestu (SI); Kroselj, Vesna, 8310 Senjernej (SI)
(74) Representative: UEXKÜLL & STOLBERG

(57) **Abstract**

The present invention relates to a solid pharmaceutical composition comprising (a) candesartan or a salt, solvate or ester thereof, (b) a polymeric agent comprising a copolymer of polyvinyl alcohol and polyethylene glycol, and (c) optionally at least one other excipient as well as a process for preparing the composition.

## Description

The present invention relates to a pharmaceutical composition which comprises 2-Ethoxy-1-[[2'-(1*H*-tetrazol-5-yl)[1,1'-biphenyl]-4-yl]methyl]-1*H*-benzimidazole-7-carboxylic acid, in the following referred to by its generic name "candesartan", or a pharmaceutically acceptable form thereof such as its 1-[[(cyclohexyloxy)carbonyl]oxy]ethyl ester, i.e. candesartan cilexetil.

Candesartan is a selective angiotensin II type-1 receptor antagonist (AT₁-antagonist). It falls in the class of drugs of benzimidazole-7-carboxylic acids and derivatives thereof. These agents exhibit a strong hypotensive action. Moreover, they are more effective and less likely to cause side effects such as cough compared to the class of ACE inhibitors.

In general, the formulation of candesartan or its pharmaceutically acceptable forms for effective oral administration to a subject has hitherto been complicated by the unique physical and chemical properties of the compound. It is known that candesartan or pharmaceutically acceptable forms thereof are stable against elevated temperature, moisture and light when they are stored individually in the solid state. However, when they are incorporated into a pharmaceutical composition decomposition of the active ingredient is observed. Up to now, this stability problem was tried to be solved by the addition of a number of different materials or by applying a number of different ways how to formulate the composition.

EP 546 358 B1 teaches to suppress decomposition of the active ingredient by providing a pharmaceutical composition comprising an oily substance. The oily substance is described to have a melting point of about 20 to 90°C and is selected from hydrocarbon, higher fatty acid, higher alcohol, fatty acid ester of polyhydric alcohol, higher alcohol ether of polyhydric alcohol and a polymer or copolymer of alkylene oxide. The copolymer of alkylene oxide is reported to be a copolymer of two or more monomers selected from ethylene oxide, propylene oxide, trimethylene oxide and tetrahydrofuran having a molecular weight of 1,000 to 10,000.

WO 2005/070398 A2 discloses a process for preparing a stable pharmaceutical composition comprising dispering candesartan cilexetil in a solution comprising a co-solvent and water to form a dispersion, granulating a blend of diluents and disintegrant with the dispersion, lubricating the granules and compressing the lubricated granules into tablets.

WO 2005/079751 A2 discloses a pharmaceutical formulation containing candesartan cilexetil and one or more fatty acids such as lipids, e.g. fatty acids and fatty acid esters, and phospholipids, e.g. phosphoglycerids and shingolipids.

WO 2006/074218 A2 describes a nanoparticulate composition comprising candesartan cilexetil particles having an average size of less than about 2,000 nm and at least one surface stabilizer.

WO 2005/084648 A1 discloses the use of water-soluble polymers to form a stable pharmaceutical composition of candesartan cilexetil. Suitable water-soluble polymers are described to include polyvinyl alcohol, maltodextrin, xanthan gum, polyvinylpyrrolidone, hydroxypropyl cellulose, hydroxypropyl methylcellulose, hydroxyethyl cellulose, sodium carboxymethylcellulose, calcium carboxymethylcellulose, methyl cellulose, tragacanth gum, agar, gellan gum, karaya gum, alginic acids, pectins, pregelatinized starch and mixtures thereof.

WO 2006/079496 A1 discloses a solid pharmaceutical composition comprising candesartan cilexetil and a hydrophilic substance with hydrocolloidal properties such as carrageenan as a stabilizing excipient. Further, WO 2006/079496 A1 discloses that candesartan cilexetil can be de-stabilized during compression molding of tablets. For this reason, the main pressure during compression molding is described to be at maximum 20 kN, preferably 10 kN.

US 2007/0014864 A, US 2007/0014853 A and US 2007/0014854 A disclose pharmaceutical granules containing an active substance having a poor water solubility such as candesartan and at least one pharmaceutically acceptable sugar.

In commercially available compositions, candesartan cilexetil is most commonly stabilized by the use of an oily substance as described in EP 546 358 B1. However, the use of such oily substances has an adverse impact on the processability of the composition as the oily substance can induce sticking of the tableting mixtures onto the punches. This can result in damages of the tablet surface and variations of the tablet mass and drug dose. In addition, handling and storage properties of such composition can also be affected.

Thus, there is a need for a pharmaceutical composition of candesartan which has a high chemical and physical stability as well as appropriate processability and compression properties avoiding the above mentioned problems associated with the use of an oily substance. Moreover, such a composition should have an appropriate disintegration time, should not require the use of expensive excipients and should be obtainable by a simple and quick process, such as wet or dry granulation, which increases the uniformity of the active ingredient distribution in the formulation, enhances the flow rates and rate uniformity, improves the physical characteristics, reduces dust, and improves the appearance of the solid dosage form. In addition, the active substance used in such a composition should have an appropriate particle size distribution and an appropriate dissolution profile and bioavailability.

These objects are surprisingly solved by the solid pharmaceutical composition according to claims 1 to 14. The invention also relates to the process according to claims 15 and 16.

The solid pharmaceutical composition according to the invention comprises
(a) candesartan or a salt, solvate or ester thereof,
(b) a polymeric agent comprising (b1) a copolymer of polyvinyl alcohol and polyethylene glycol, and
(c) optionally at least one other excipient.

The component (a) of the composition is candesartan or a salt, solvate or ester thereof. The candesartan can be used in different polymorphic or pseudopolymorphic forms such as solvates like hydrates. In addition, the candesartan can also be present in the form of a salt thereof. Examples of useful salts include addition salts with an inorganic base such as sodium hydroxide or with an organic base such as an amine. Moreover, candesartan can be present in the form of an ester. Preferably, candesartan is used in the form of an ester and more preferably candesartan cilexetil is used.

It is preferred that the composition comprises from 3 to 30 wt.-%, more preferably 4 to 20 wt.-% and most preferably 5 to 10 wt.-% of candesartan or salt or solvate or ester thereof. It is also preferred that a single dosage form of the pharmaceutical composition according to the invention comprises from 2 to 32 mg, e.g. 2, 4, 8, 16 or 32 mg, of candesartan or salt or solvate or ester thereof, calculated as candesartan.

If candesartan cilexetil is used as component (a), its average particle size is preferably ranging from 2 to 30 µm, more preferably 3 to 25 µm and most preferably 5 to 15 µm. In a preferred embodiment, the candesartan cilexetil is characterized by the following particle size distribution:

| **D[4,3]** (average particle size) | **13µm** |
|---|---|
| d10 | 2, 3µm |
| d50 | 7, 5µm |
| d90 | 26, 6µm |

The average particle size can be determined by laser light scattering using e.g. a Malvern-Mastersizer Apparatus with Isopar-L as dilution medium. Particle sizes are determined by measuring the angular distribution of laser light scattered by a homogeneous suspension of particles after 1 minute of sonication.

The component (b) of the composition is a polymeric agent which comprises (b1) a copolymer of polyvinyl alcohol and polyethylene glycol (PVA/PEG copolymer). As used herein, the term "copolymer" denotes a polymer of two or more different monomers. Accordingly, a copolymer of polyvinyl alcohol and polyethylene glycol is a molecule of high relative molecular mass which is composed of repeating structural units derived from vinyl alcohol and ethylene glycol, which units are connected by covalent chemical bonds.

It has surprisingly been found that by using candesartan in combination with the polymeric agent (b) a highly stable pharmaceutical composition can be prepared even without adding any oily substance as described in EP 546 358 B1. The stability of the composition according to the invention is comparable or even higher than that of compositions comprising an oily substance. As used herein, the term "highly stable" means that the increase of the total content of impurities in dosage forms packed in either PVC or PVC/PVDC blisters is less than 0.3% after 3 months storage at 45°C and 75% relative humidity. Moreover the use of the combination of candesartan and polymeric agent is advantageous since the polymeric agent does not have an adverse effect on the processability of the composition.

It is preferred that the copolymer (b1), i.e. the PVA/PEG copolymer, is a grafted copolymer. The term "grafted copolymer" means that the copolymer is in the form of a branched copolymer in which the side chains are structurally distinct from the main chain. In particular, the grafted PVA/PEG copolymer (b1) is a molecule having a polyethylene glycol main chain and several polyvinyl alcohol side chains.

The weight ratio between the polyethylene glycol units and the polyvinyl alcohol units preferably ranges from 1:20 to 5:1, more preferably 1:10 to 1:1 and most preferably is about 1:3.

Accordingly, it is particular preferred to use a copolymer (b1) which is a grafted PVA/PEG copolymer having a polyethylene glycol main chain and polyvinyl alcohol side chains, wherein the weight ratio of the polyethylene glycol units to the polyvinyl alcohol units is about 1:3.

In particular, the copolymer (b1) used in the composition according to the invention has a melting point higher than 150°C, preferably higher than 180°C, more preferably higher than 200°C and most preferably has a melting point of about 209°C.

Furthermore, it is preferred that the copolymer (b1) has a molecular weight more than 30,000, preferably more than 35,000, more preferably more than 40,000 and most preferably has a molecular weight of about 45,000 daltons as determined by gel permeation chromatography (GPC).

In a preferred embodiment of the invention, the polymeric agent (b) further comprises (b2) polyvinyl alcohol, i.e. free polyvinyl alcohol which does not form part of the polyvinyl alcohol-polyethylene glycol copolymer (b1).

It is advantageous that the polyvinyl alcohol (b2) has a molecular weight more than 20,000, preferably more than 30,000 and most preferably has a molecular weight of about 35,000 daltons as determined by gel permeation chromatography (GPC).

In a particularly preferred embodiment, the polymeric agent (b) comprises, based on the total amount of the polymeric agent,
(b1) 40 to 80 wt.-%, preferably 55 to 65 wt.-% and more preferably about 60 wt.-% of polyvinyl alcohol-polyethylene glycol copolymer,
(b2) 20 to 60 wt.-%, preferably 35 to 45 wt.-% and more preferably about 40 wt.-% of polyvinyl alcohol, and
(b3) optionally 0.1 to 1 wt.-%, preferably 0.1 to 0.3 wt.-% and more preferably about 0.3 wt.-% of silicon dioxide.

A suitable commercially available polymeric agent (b) is Kollicoat^{®} Protect which is sold by BASF AG as film-former for film coatings.

The weight ratio of (a) candesartan, a salt, solvate or ester thereof to (b) the polymeric agent can range from 1:4 to 4:1, preferably ranges from 1:3 to 3:1, more preferably 1:2 to 2:1 and most preferably is about 1:1 to 2:1. These specific ratios result in that the dissolution of the active ingredient from the composition according to the invention is increased compared to compositions comprising other or even no amounts of polymeric agent (b).

The other excipient (c) of the composition of the invention can be selected from pharmaceutically acceptable diluents, binders, disintegrants, surfactants, lubricants, pigments, colorants and mixtures thereof.

Suitable examples of diluents include but are not limited to lactose, mannitol, sorbitol, starch, microcrystalline cellulose, sucrose, fructose, calcium phosphate, calcium sulphate and amylose. The preferred diluent is lactose, which can be in anhydrous or hydrous form, for example lactose monohydrate.

The binder can be selected from the group consisting of glucose, acacia, tragacanth, sucrose, gelatin, starch, pregelatinized starch, polyvinylpyrrolidone, polymethacrylates, cellulose derivatives such as methylcellulose, ethylcellulose, hydroxypropyl cellulose, hydroxypropyl methylcellulose, hydroxyethyl cellulose, sodium carboxymethylcellulose and mixtures thereof.

Examples of suitable disintegrants include starch, pregelatinized starch, sodium starch glycolate, alignates, polacrylinate potassium, crospovidone, clays, cellulose derivatives such as low-substituted hydroxypropyl cellulose, hydroxypropyl methylcellulose, carmellose sodium, carmellose calcium and mixtures thereof.

Examples of suitable surfactants are those having a melting point of more than 120°C, preferably more than 150°C, most preferably more than 170°C, such as sodium dodecyl sulphate. In one embodiment, surfactants are used in an amount of 2 to 50 wt.-%, preferably 4 to 40 wt.-% and most preferably 5 to 20 wt.-%, based on the weight of candesartan or salt, solvate or ester thereof such as candesartan cilexetil.

Examples of suitable lubricants are magnesium stearate, magnesium lauryl sulphate, sodium lauryl sulphate, sodium stearyl fumarate, hydrogenated vegetable oil and stearic acid. Preferably, magnesium stearate is used.

The composition can also comprise pigments or colorants such as metal oxides like iron or titanium oxides. Pigments and/or colorants can be incorporated exclusively intra or extra granular, for example in an optional coating of a core, or can be divided among the mentioned phases.

In a particular preferred embodiment, the composition accoding to the invention independently comprises, on the basis of the weight of the total composition,
(a) 3 to 30 wt.-%, more preferably 4 to 20 wt.-% and most preferably 5 to 10 wt.-% of candesartan, a salt, solvate or ester thereof,
(b) 1 to 30 wt.-%, preferably 2 to 20 wt.-%, more preferably 3 to 15 wt.-% and most preferably 4 to 10 wt.-% of the polymeric agent,
(c) optionally 20 to 85 wt.-%, preferably 35 to 80 wt.-%, and most preferably 50 to 75 wt.-% of a diluent,
(d) optionally 0.5 to 25 wt.-%, preferably 1 to 20 wt.-%, more preferably 1.5 to 15 wt.-% and most preferably 2 to 13 wt.-% of a binder,
(e) optionally 0.5 to 20 wt.-%, preferably 1 to 15 wt.-%, more preferably 1.5 to 10 wt.-% and most preferably 2 to 5 wt.-% of a disintegrant, and
(f) optionally 0.1 to 2 wt.-%, preferably 0.15 to 1 wt.-% and most preferably 0.2 to 0.4 wt.-% of a lubricant.

In a further embodiment, the composition can comprise inert cores. As used herein, the term "inert cores" denotes particles which do not contain candesartan or a salt, solvate or ester thereof. The inert cores can also be referred to as base granules, inert granules or placebo granules. Optionally, the inert cores comprise a part or the entire amount of the above mentioned diluent, binder and/or disintegrant.

The composition according to the invention is preferably in the form of granules or tablets prepared from granules.

The pharmaceutical composition of the present invention can also comprise (a) candesartan or a salt, solvate or ester thereof in combination with at least one other active ingredient agent selected from the group of antihypertensives, diuretics, lipid regulators or antidiabetics.

Antihypertensive can be selected from the group of angiotensive converting enzyme (ACE) inhibitors, angiotensin receptor blockers (ARBs), calcium channel blockers (CCBs) and antiadrenergics. Lipid regulators can for example be HMG CoA reductase inhibitors.

It has surprisingly been found that the stability of the candesartan composition according to the invention is not adversely affected if the other active ingredient is selected from the group consisting of captopril, enalapril, cilazapril, lisinopril, trandolapril, ramipril, fosinopril, perindopril, amlodipine, diltiazem, felodipine, nifedipine, nitrendipine, verapamil, acebutol, atenolol, betaxolol, bisoprolol, metoprolol, carvedilol, lovastatin, simvastatin, pravastatin, atorvastatin, fluvastatin, rosuvastatin, indapamide, hydrochlorothiazide, sulfonyl urea, metglinides such as nateglinide or repaglinide, thiazolidinediones such as pioglitazone or rosiglitazone, α-glucosidase inhibitors, incretin mimetics, biguanides such as metformin hydrochloride and pharmaceutically acceptable salts thereof.

The invention also relates to a process for preparing the pharmaceutical composition comprising
(i) converting a suspension of (a) candesartan, a salt, solvate or ester thereof in a solution comprising the polymeric agent (b), optionally a binder which can be selected from hydroxypropyl cellulose, hydroxypropyl methylcellulose or povidone and optionally a surfactant, into a solid form,
(ii) optionally adding a diluent, a disintegrant, a binder and/or a lubricant to the solid form obtained in step (i), and
(iii) optionally compressing the mixture of step (ii) to obtain tablets.

In step (i), a suspension of candesartan, a salt, solvate or ester thereof is provided in a solution of the polymeric agent (b). This suspension is granulated, i.e. converted into a solid form. Such conversion can be performed by granulating a mixture of excipients with a dispersion obtained by suspending candesartan or a salt, solvate or ester thereof in a solution of the polymeric agent and optionally a binder and surfactant. According to another embodiment, the conversion into the solid form is performed by coating (e.g. spray coating) the suspension onto inert cores which can be inert pellets or inert granules. Inert granules can be obtained by processes known in the state of the art, like agglomerating pharmaceutically acceptable excipients such as diluents, binders and disintegrants in a high shear granulator or fluid bed granulator and drying and sieving the obtained granulate.

Then, in step (ii) the obtained solid form is optionally dried and mixed with further excipients such as diluents, disintegrants, binders and/or lubricants.

Finally, the obtained mixture of step (ii) can be compressed to obtain tablets.

The invention is in the following further illustrated by reference to the following examples.

### Examples

### Example 1

| | **Example 1** |
|---|---|
| ***ingredients of granulate*** | mg/tablet |
| candesartan cilexetil | 32.0 |
| lactose | 345.0 |
| maize starch | 69.5 |
| calcium carboxymethyl-cellulose (calcium CMC) | 8.0 |
| hydroxypropyl cellulose (Klucel LF) | 12.0 |
| PVA/PEG copolymer (Kollicoat Protect) | 16.0 |
| purified water | q.s. |

| ***extra granular*** | |
|---|---|
| calcium CMC | 8.0 |
| Mg stearate | 1.5 |
| **Tablet weight** | **492.0** |
| **Punch** | circular, 11mm |
| **Hardness (N)** | 54-69 |
| **Friability (%)** | nil |
| **Thickness (mm)** | 5.30-5.36 |
| **Disintegration time (min)** | 7-18 |

Lactose, calcium carboxymethylcellulose (calcium CMC) and maize starch were sieved through a 40 Mesh sieve and mixed in a high shear granulator. The obtained powder mixture was granulated with a dispersion obtained by suspending candesartan cilexetil in a solution of Kollicoat Protect and Klucel LF. The obtained granulate was dried in a fluid bed dryer to a water content below the LOD (2 wt.-%). The obtained dry granulate was sieved and mixed with the extra granular ingredients and compressed into tablets.

### Examples 2 to 4

| | **Example 2** | **Example 3** | **Example 4** |
|---|---|---|---|
| ***ingredients of granulate*** | mg/tablet | | |
| Candesartan cilexetil | 32.0 | 32.0 | 32.0 |
| Manitol | 270.0 | 270.0 | 270.0 |
| Sodium CMC | 8.0 | 8.0 | 8.0 |
| Hydroxypropyl methylcellulose (Hypromellose 3 cps) | 8.0 | - | - |
| Povidone (Kollidon K30) | - | 8.0 | 8.0 |
| PVA/PEG copolymer (Kollicoat Protect) | 16.0 | 16.0 | 16.0 |
| Sodium lauryl sulphate | 3.2 | 3.2 | 3.2 |
| Purified water | q.s. | q.s. | q.s. |

| ***Extra granular*** | | | |
|---|---|---|---|
| Sodium CMC | 8.0 | 8.0 | 8.0 |
| Microcrystalline cellulose | 132.3 | 132.3 | 144.3 |
| Mg stearate | 1.5 | 1.5 | 1.5 |
| **Tablet weight** | **480.0** | **480.0** | **492.0** |
| **Punch** | oblong 13x8 mm | oblong 13x8 mm | oblong 15x8 mm |

Manitol and sodium CMC were sieved through a 40 Mesh sieve and mixed in high shear granulator. The obtained powder mixture was granulated with a dispersion obtained by suspending candesartan cilexetil in a solution of Kollicoat Protect, sodium lauryl sulphate and binder (either Kollidon K30 or hypromellose 3 cps). The obtained granulate was dried in a fluid bed dryer to a water content below the LOD (2 wt.-%). The obtained dry granulate was sieved and mixed with the extra granular ingredients and compressed into tablets.

### Examples 5 to 7

| | **Example 5** | **Example 6** | **Example 7** |
|---|---|---|---|
| ***ingredients of granulate*** | mg/tablet | | |
| candesartan cilexetil | 32.0 | 32.0 | 32.0 |
| sorbitol | 30.0 | 30.0 | 30.0 |
| manitol | 240.0 | 240.0 | 240.0 |
| Crospovidone* | 8.0 | 8.0 | 8.0 |
| Povidone | 10.0 | 8.0 | 8.0 |
| PVA/PEG copolymer (Kollicoat Protect) | 32.0 | 32.0 | 32.0 |
| sodium lauryl | 4.8 | 3.2 | 3.2 |
| sulphate | | | |
| purified water | q.s. | q.s. | q.s. |

| ***extra granular*** | | | |
|---|---|---|---|
| Crospovidone ** | 8.0 | 8.0 | 8.0 |
| microcrystalline cellulose | 130.3 | 132.3 | - |
| Cellactose | - | - | 132.3 |
| Mg stearate | 1.5 | 1.5 | 1.5 |
| **Tablet weight** | **480.0** | **480.0** | **480.0** |
| **Punch** | oblong 13x8 mm | oblong 13x8 mm | oblong 13x8 mm |

| | | | |
|---|---|---|---|
| * average particle size <80µm ** average particle size >80µm | | | |

Manitol and crospovidone were sieved through a 40 Mesh sieve and transferred to fluid bed granulator equipped with top spray nozzle. The obtained powder mixture was granulated with a dispersion obtained by suspending candesartan cilexetil in a solution of Kollicoat Protect, sorbitol, sodium lauryl sulphate and Klucel LF in water with simultaneous evaporation of water. The obtained granulate was dried in a fluid bed dryer to a water content below the LOD (2 wt.-%). The obtained dry granulate was sieved and mixed with the extra granular ingredients and compressed into tablets.

### Examples 8 to 10

| | **Example 8** | **Example 9** | **Example 10** |
|---|---|---|---|
| ***ingredients of granulate*** | mg/tablet | | |
| candesartan cilexetil | 32.0 | 32.0 | 32.0 |
| lactose (Pharmatose 200M) | 317.0 | - | - |
| manitol | - | 200.0 | 220.6 |
| maize starch | 69.5 | - | - |
| microcrystalline cellulose | - | 40.0 | 40.0 |
| calcium CMC | 10.0 | - | - |
| low-substituted hydroxypropyl cellulose (L-HPC) | - | 24.0 | 24.0 |
| sodium lauryl sulphate | - | 4.8 | 4.8 |
| hydroxypropyl cellulose (Klucel LF) | 8.0 | - | - |
| hydroxypropyl methylcellulose (Hypromellose 3 cps) | - | 10.0 | 12.0 |
| PVA/PEG copolymer (Kollicoat Protect) | 32.0 | 32.0 | 48.0 |
| purified water | q.s. | q.s. | q.s. |

| ***extra granular*** | | | |
|---|---|---|---|
| calcium CMC | 10.0 | - | - |
| L-HPC | - | 24.0 | 48.0 |
| Cellactose | - | 56.0 | 69.0 |
| Mg stearate | 1.5 | 1.0 | 1.6 |
| **Tablet weight** | **480.0** | **480.0** | **500.0** |
| **Punch** | circular, 11 mm | oblong, 13x8 mm | oblong, 15x8 mm |

The composition of Example 8 was prepared according to the process of Example 1.

In order to obtain the compositions of Examples 9 and 10, manitol and L-HPC were sieved through a 40 Mesh sieve and transferred to a fluid bed granulator equipped with top spray nozzle. The obtained powder mixture was granulated with a dispersion obtained by suspending candesartan cilexetil in a solution of Kollicoat Protect, sorbitol, sodium lauryl sulphate and hypromellose in water with simultaneous evaporation of water. The obtained granulate was dried in a fluid bed dryer to a water content below the LOD (2 wt.-%). The obtained dry granulate was sieved and mixed with the extra granular ingredients and compressed into tablets.

### Examples 11 to 13

| | **Example 11 (reference)** | **Example 12** | **Example 13** |
|---|---|---|---|
| ***ingredients of granulate*** | mg/unit | | |
| ***placebo granules*** | | | |
| lactose monohydrate | 89.0 | 89.0 | 89.0 |
| maize starch | 22.5 | 22.5 | 22.5 |
| hydroxypropyl cellulose (Klucel LF) | 2.5 | 2.5 | 2.5 |
| sodium CMC | 6.0 | 6.0 | 6.0 |
| purified water | q.s. | q.s. | q.s. |

| ***coating*** | | | |
|---|---|---|---|
| candesartan cilexetil | 32.0 | 32.0 | 32.0 |
| manitol | - | 32.0 | 32.0 |
| sodium lauryl sulphate | - | 3.2 | 4.8 |
| hydroxypropyl cellulose (Hyprolose) | 4.0 | 4.0 | 4.0 |
| PVA/PEG copolymer (Kollicoat Protect) | - | 32.0 | 48.0 |
| purified water | q.s. | q.s. | q.s. |

| ***extra granular*** | | | |
|---|---|---|---|
| lactose (Pharmatose DCL 15) | 312.5 | 180.5 | 166.5 |
| Cellactose | - | 64.8 | 60.2 |
| sodium CMC | 10.0 | 10.0 | 10.0 |
| Mg stearate | 1.5 | 1.5 | 1.5 |
| **Tablet weight** | **480.0** | **480.0** | **480.0** |
| **Punch** | circular, 11 mm, plain | oblong, 13x8 mm | oblong, 13x8 mm |

### Preparation of placebo granules:

a. Lactose monohydrate, maize starch and calcium CMC were sieved through a 40 Mesh sieve and mixed in a rapid mixer granulator for 5 minutes.
b. Klucel LF was dissolved in water to get a clear solution.
c. The blend obtained in step a. is granulated in a rapid mixer granulator with the Klucel LF solution obtained in step b. for 2-3 minutes at low speed to obtain a wet granular mass.
d. The wet granules were dried in a fluid bed dryer to achieve a water content a water content below the LOD (2 wt.-%). The dried granules were sized by sieving them through a 24 Mesh sieve.

### Coating of placebo granules:

Candesartan cilexetil was suspended in a solution obtained by dissolving the rest of the coating ingredients in purified water. Placebo granules were coated in a fluid bed dryer equipped with spray nozzle inserted at the bottom of the product container. The spray rate inlet air temperature and flow rate were adjusted so that secondary agglomeration of placebo granules was minimized (<3 wt.-%). The coated granules were dried and sieved.

### Lubrication and tablet compression:

Subsequently, the dried coated granules were mixed with the extra granular excipients and the obtained mixture was compressed into tablets.

### Examples 14 and 15

| | **Example 14** | **Example 15** |
|---|---|---|
| ***ingredients of granulate*** | mg/unit | |
| ***placebo granules*** | | |
| lactose monohydrate (Pharmatose 200 M) | 163.5 | 163.5 |
| maize starch | 41.0 | 41.0 |
| hydroxypropyl cellulose (Klucel LF) | 4.5 | 4.5 |
| calcium CMC | 11.0 | 11.0 |
| purified water | q.s. | q.s. |

| ***coating*** | | |
|---|---|---|
| candesartan cilexetil | 32.0 | 32.0 |
| hydroxypropyl cellulose (Klucel HF) | 4.0 | 4.0 |
| PVA/PEG copolymer (Kollicoat Protect) | 32.0 | 16.0 |
| purified water | q.s. | q.s. |

| ***extra granular*** | | |
|---|---|---|
| lactose (Pharmatose DCL 15) | 180.5 | 196.5 |
| calcium CMC | 10.0 | 10.0 |
| Mg stearate | 1.5 | 1.5 |
| **Tablet weight** | **480.0** | **480.0** |
| **Punch** | circular, 11 mm, embossed | circular, 11 mm, embossed |

### Preparation of Placebo granules:

a. Pharmatose 200 M, maize starch and calcium CMC were sieved and loaded in a rapid mixer granulator and mixed for 5 minutes.
b. Klucel LF was dissolved in water. The obtained solution was mixed with the blend in the rapid mixer granulator. The wet mass was dried in a fluid bed dryer to a water content below the LOD (2 wt.-%). The dried granules were passed through a 1 mm sieve.

### Preparation of coating suspension:

a. Kollicoat Protect was dissolved in purified water to get a clear solution. Then Klucel LF was dissolved in this solution.
b. Candesartan cilexetil was added gradually to this solution while continuously stirring to form a smooth suspension.
c. The resulting suspension was used for coating placebo granules in a fluid bed processor.

### Spray Granulation:

a. The placebo granules were taken for coating process in a fluid bed processor.
b. The coating was performed by bottom spray wurster arrangement. Once the coating process was completed, the drug coated granules were dried in a mini-Glatt for 10-15 minutes to obtain a water content below the LOD (2 wt.-%).
c. The dried granules were sieved through a sieve with 750µm openings.

### Lubrication:

The extra granular excipients Pharmatose DCL 15 and calcium CMC were mixed with the drug coated granules in a drum blender for 15 minutes. Further, the granules were lubricated with magnesium stearate by mixing for additional 2 minutes. The obtained granules were used for compression.

### Tablet compression:

The lubricated blend was compressed using 11 mm punch.

### Example 16

| | **Example 16** |
|---|---|
| ***ingredients of granulate*** | mg/unit |
| ***placebo granules*** | |
| lactose monohydrate (Pharmatose 200 M) | 165.0 |
| maize starch | 40.0 |
| hydroxypropyl cellulose (Klucel LF) | 4.5 |
| calcium CMC | 10.0 |
| Iron oxyde | 0.5 |
| purified water | q.s. |

| ***coating*** | |
|---|---|
| candesartan cilexetil | 32.0 |
| hydroxypropyl cellulose (Klucel HF) | 4.0 |
| PVA/PEG copolymer (Kollicoat Protect) | 16.0 |
| Iron oxyde | 0.5 |
| purified water | q.s. |

| ***extra granular*** | |
|---|---|
| lactose (Pharmatose DCL 15) Iron | 196.5 |
| calcium CMC | 10.0 |
| Mg stearate | 0.5 |
| oxyde | 0.5 |
| **Tablet weight** | **480.0** |
| **Punch** | circular, 11 mm, embossed |

The composition of Example 16 was prepared according to the process of Example 14.

### Example 17 - Comparison of dissolution profile

The *in vitro* dissolution profile of candesartan celexetil from tablets according to Example 14 was measured (900 ml of dissolution medium: 0.7% polysorbate 20 in phosphate buffer 0.05 M (pH 6.5), apparatus II according to USP XXIII (paddle)) and compared in the following table with the dissolution profile of candesartan cilexetil from a reference tablet using polyethylene glycol (Macrogol 8000) as a stabilizing oily substance.

| **Time (min)** | **Reference** | | **Example 14** | |
|---|---|---|---|---|
| | Average wt.-% of released candesartan (6 tablets) | RSD | Average wt.-% of released candesartan (6 tablets) | RSD |
| **0** | **0** | 0 | **0** | 0 |
| **5** | **14.1** | 15.3 | **15.6** | 36.7 |
| **10** | **33.2** | 14.6 | **40.3** | 18.9 |
| **15** | **45.5** | 11.5 | **65.3** | 13.0 |
| **30** | **85.9** | 5.8 | **92.6** | 3.4 |
| **45** | **95.7** | 2.8 | **97.3** | 3.9 |
| **60** | **98.3** | 3.0 | **103.0** | 1.8 |

It can be seen from the table that after 15 minutes 65 wt.-% of candesartan cilexetil is released from the composition according to the invention, while from the reference composition only 45 wt.-% is released. Thus, the dissolution of the active ingredient is even improved when using a combination of candesartan (a) and polymeric agent (b).

### Example 18 - Tablets comprising a combination of candesartan cilexetil and hydrochlorothiazide (HCTZ)

| | **Example 18** |
|---|---|
| ***ingredients of granulate*** | mg/tablet |
| candesartan cilexetil | 16.0 |
| lactose monohydrate | 120.0 |
| maize starch | 41.0 |
| calcium CMC | 6.0 |
| hydroxypropyl cellulose (Klucel HF) | 10.0 |
| Kollicoat Protect | 24.0 |
| purified water | q.s. |

| ***extra granular*** | |
|---|---|
| hydrochlorothiazide | 12.5 |
| Cellactose 80 | 41.5 |
| calcium CMC | 6.0 |
| Mg stearate | 3.0 |
| **Tablet weight** | **280.0** |
| **Punch** | Round, 9 mm |

Lactose, calcium CMC and maize starch were sieved through a 40 Mesh sieve and mixed in a high shear granulator. The obtained powder mixture was granulated with a dispersion obtained by suspending candesartan cilexetil in a solution of Kollicoat Protect and Klucel LF. The obtained granulate was dried in a fluid bed dryer to a water content below the LOD (2 wt.-%). The obtained granulate was dried, sieved and mixed with the rest of ingredients and compressed into tablets using round punches.

### Example 19 - Tablets comprising a combination of candesartan cilexetil and indapamide

| | **Example 19** |
|---|---|
| ***ingredients of granulate*** | mg/tablet |
| candesartan cilexetil | 16.0 |
| indapamide | 1.25 |
| lactose monohydrate | 103.75 |
| maize starch | 60.0 |
| croscarmelose sodium | 4.0 |
| hydroxypropyl cellulose (Klucel HF) | 7.0 |
| Kollicoat Protect | 28.0 |
| Purified water | q.s. |

| ***extra granular*** | |
|---|---|
| lactose monohydrate DC* | 41.5 |
| croscamellose sodium | 4.0 |
| Mg stearate | 2.5 |
| **Tablet weight** | **268.0** |
| **Punch** | Round, 9 mm |

| | |
|---|---|
| * direct compression grade | |

Indapamide, lactose monohydrate, croscarmelose sodium and maize starch were sieved through a 40 Mesh sieve and mixed in a high shear granulator. The obtained powder mixture was granulated with a dispersion obtained by suspending candesartan cilexetil in a solution of Kollicoat Protect and Klucel LF. The obtained granules were dried, sieved and mixed with the rest of the ingredients and compressed into tablets using round punches.

### Example 20 - Tablets comprising a combination of candesartan cilexetil and atorvastatin calcium

| | **Example 20** |
|---|---|
| ***ingredients of granulate*** | mg/tablet |
| ***granulate 1*** | |
| candesartan cilexetil | 16.0 |
| lactose monohydrate | 105.0 |
| maize starch | 60.0 |
| croscarmelose sodium | 4.0 |
| hydroxypropyl cellulose (Klucel HF) | 7.0 |
| Kollicoat Protect | 28.0 |
| purified water | q.s. |

| ***granulate 2*** | |
|---|---|
| atorvastatin calcium | 20.72 |
| lactose monohydrate | 111.68 |
| sodium lauryl sulfate | 2.0 |
| sodium hydroxide | 1.0 |
| hydroxypropyl cellulose (Klucel HF) | 5.0 |
| methanol | q.s. |

| ***extra granular*** | |
|---|---|
| croscamellose sodium | 6.0 |
| Mg stearate | 4.0 |
| **Tablet weight** | **370 mg** |
| **Punch** | Round, 10 mm |

Lactose monohydrate, croscarmelose sodium and maize starch were sieved and mixed in a fluid bed granulator. The obtained powder mixture was granulated with a dispersion obtained by suspending candesartan cilexetil in a solution of Kollicoat Protect and Klucel LF. The obtained granules 1 were dried and sieved. Atorvastatin calcium, sodium hydroxide, sodium lauryl sulfate and Klucel EF were dissolved in methanol and sprayed onto lactose monohydrate in a fluid bed dryer. The obtained granules 2 were dried and sieved. Both granulates were mixed, croscarmellose sodium and magnesium stearate were admixed and the obtained mixture was compressed into tablets using round punches. The obtained tablets were coated using PVA based Opadry coating and packed into Alu/Alu blisters under an atmosphere with reduced oxygen content.

### Example 21 - Tablets comprising a combination of candesartan cilexetil and amlodipin besilate

| | **Example 21** |
|---|---|
| ***ingredients of granulate*** | mg/tablet |
| candesartan cilexetil | 16.0 |
| mannitol | 106.0 |
| microcrystalline cellulose | 35.0 |
| croscarmelose sodium | 4.0 |
| hydroxypropyl cellulose (Klucel HF) | 7.0 |
| Kollicoat Protect | 32.0 |
| purified water | q.s. |

| ***extra granular*** | |
|---|---|
| amlodipine besilate | 6.94 |
| microcrystalline cellulose | 110.06 |
| starch pregelatinized | 25.0 |
| sodium starch glycolate | 18.0 |
| Mg stearate | 3.0 |
| **Tablet weight** | **363.0 mg** |
| **Punch** | Round, 10 mm |

Mannitol, microcrystalline cellulose and croscarmelose sodium were sieved and mixed in a fluid bed granulator. The obtained powder mixture was granulated with a dispersion obtained by suspending candesartan cilexetil in a solution of Kollicoat Protect and Klucel LF. The obtained granules were dried and sieved. The other ingredients were admixed and the obtained mixture was compressed into tablets.

## Claims

1. Solid pharmaceutical composition comprising
(a) candesartan or a salt, solvate or ester thereof,
(b) a polymeric agent comprising (b1) a copolymer of polyvinyl alcohol and polyethylene glycol, and
(c) optionally at least one other excipient.

2. Composition according to claim 1, which comprises (a) candesartan cilexetil.

3. Composition according to claim 1 or claim 2, wherein the copolymer (b1) is a grafted copolymer.

4. Composition according to claim 3, wherein the grafted copolymer has a polyethylene glycol main chain and polyvinyl alcohol side chains, wherein the weight ratio of the polyethylene glycol units to the polyvinyl alcohol units preferably ranges from 1:20 to 5:1, more preferably 1:10 to 1:1 and most preferably is about 1:3.

5. Composition according to any one of claims 1 to 4, wherein the copolymer (b1) has a melting point higher than 150°C, preferably higher than 180°C, more preferably higher than 200°C and most preferably has a melting point of about 209°C.

6. Composition according to any one of claims 1 to 5, wherein the copolymer (b1) has a molecular weight of more than 30,000, preferably more than 35,000, more preferably more than 40,000 and most preferably has a molecular weight of about 45,000.

7. Composition according to any one of claims 1 to 6, wherein the polymeric agent further comprises (b2) polyvinyl alcohol.

8. Composition according to claim 7, wherein the polymeric agent (b) comprises based on the total amount of the polymeric agent,
(b1) 40 to 80 wt.-%, preferably 55 to 65 wt.-% and more preferably about 60 wt.-% of polyvinyl alcohol-polyethylene glycol copolymer,
(b2) 20 to 60 wt.-%, preferably 35 to 45 wt.-% and more preferably about 40 wt.-% of polyvinyl alcohol, and
(b3) optionally 0.1 to 1 wt.-%, preferably 0.1 to 0.3 wt.-% and more preferably about 0.3 wt.-% of silicon dioxide.

9. Composition according to claim 7 or 8, wherein the polyvinyl alcohol (b2) has a molecular weight of more than 20,000, preferably more than 30,000 and most preferably has a molecular weight of about 35,000.

10. Composition according to any one of claims 1 to 9, wherein the weight ratio of (a) candesartan, a salt, solvate or ester thereof to (b) the polymeric agent ranges from 1:4 to 4:1, preferably 1:3 to 3:1, more preferably 1:2 to 2:1 and most preferably is about 1:1 to 2:1.

11. Composition according to any one of claims 1 to 10, wherein the other excipient (c) is a diluent, binder, disintegrant, surfactant, lubricant, pigment, colorant or a mixture thereof.

12. Composition according to any one of claims 1 to 11, which independently comprises on the basis of the weight of the total composition,
(a) 3 to 30 wt.-%, more preferably 4 to 20 wt.-% and most preferably 5 to 10 wt.-% of candesartan, a salt, solvate or ester thereof,
(b) 1 to 30 wt.-%, preferably 2 to 20 wt.-%, more preferably 3 to 15 wt.-% and most preferably 4 to 10 wt.-% of the polymeric agent,
(c) optionally 20 to 85 wt.-%, preferably 35 to 80 wt.-%, and most preferably 50 to 75 wt.-% of a diluent,
(d) optionally 0.5 to 25 wt.-%, preferably 1 to 20 wt.-%, more preferably 1.5 to 15 wt.-% and most preferably 2 to 13 wt.-% of a binder,
(e) optionally 0.5 to 20 wt.-%, preferably 1 to 15 wt.-%, more preferably 1.5 to 10 wt.-% and most preferably 2 to 5 wt.-% of a disintegrant, and
(f) optionally 0.1 to 2 wt.-%, preferably 0.15 to 1 wt.-% and most preferably 0.2 to 0.4 wt.-% of a lubricant.

13. Composition according to any one of claims 1 to 12, which comprises inert cores.

14. Composition according to any one of claims 1 to 13, which further comprises an active ingredient selected from the group consisting of captopril, enalapril, cilazapril, lisinopril, trandolapril, ramipril, fosinopril, perindopril, amlodipine, diltiazem, felodipine, nifedipine, nitrendipine, verapamil, acebutol, atenolol, betaxolol, bisoprolol, metoprolol, carvedilol, lovastatin, simvastatin, pravastatin, atorvastatin, fluvastatin, rosuvastatin, indapamide, hydrochlorothiazide, sulfonyl urea, metglinides such as nateglinide or repaglinide, thiazolidinediones such as pioglitazone or rosiglitazone, α-glucosidase inhibitors, incretin mimetics, biguanides such as metformin hydrochloride and pharmaceutically acceptable salts thereof.

15. Process for preparing a composition according to any one of claims 1 to 14 comprising
(i) converting a suspension of (a) candesartan, a salt, solvate or ester thereof in a solution comprising the polymeric agent (b), optionally a binder which can be selected from hydroxypropyl cellulose, hydroxypropyl methylcellulose or povidone and optionally a surfactant, into a solid form,
(ii) optionally adding a diluent, a disintegrant, a binder and/or a lubricant to the solid form obtained in step (i), and
(iii) optionally compressing the mixture of step (ii) to obtain tablets.

16. Process according to claim 15, wherein in step (i) inert cores are coated with the suspension of candesartan, a salt, solvate or ester thereof.
